Europäisches Patentamt

European Patent Office

Office européen des brevets

(19)

(11) Veröffentlichungsnummer: **0 031 547**
**B1**

# (12) EUROPÄISCHE PATENTSCHRIFT

(45) Veröffentlichungstag der Patentschrift:
14.03.84

(21) Anmeldenummer: 80107970.8

(22) Anmeldetag: 17.12.80

(51) Int. Cl.³: **B 65 D 6/06,** B 65 D 75/36,
A 61 J 1/00

(54) Verpackungsbehälter insbesondere für Medikamente.

(30) Priorität: 28.12.79 DE 2952616

(43) Veröffentlichungstag der Anmeldung:
08.07.81 Patentblatt 81/27

(45) Bekanntmachung des Hinweises auf die Patenterteilung:
14.03.84 Patentblatt 84/11

(84) Benannte Vertragsstaaten:
CH FR GB IT LI NL

(56) Entgegenhaltungen:
DE - A - 2 300 834
DE - B - 2 013 868
FR - A - 1 560 591
US - A - 2 834 456
US - A - 2 985 296
US - A - 3 049 224
US - A - 3 157 280

(73) Patentinhaber: **Artusi, Aldo, Blautraubenstrasse 25,
CH-8200 Schaffhausen (CH)**

(72) Erfinder: **Artusi, Aldo, Blautraubenstrasse 25,
CH-8200 Schaffhausen (CH)**

(74) Vertreter: **Hiebsch, Gerhard F., Dipl.-Ing.,
Erzbergerstrasse 5A Postfach 464, D-7700 Singen 1 (DE)**

BUNDESDRUCKEREI BERLIN

Verpackungsbehälter, insbesondere für Medikamente

Die Erfindung betrifft einen Verpackungsbehälter mit einem Aufnahmeteil für Füllgüter, insbesondere für Medikamente od. dgl., welche gegebenenfalls in einem Medikamentengefäß untergebracht sind, und einem Deckel, der mittels in seitliche Nuten eingreifender Führungsstreifen am Aufnahmeteil verschiebbar ist, wobei an einander gegenüberliegenden Enden des Aufnahmeteils und/oder des Deckels die Verschiebbarkeit des Deckels verhindernde seitlich überstehende und abbiegbare Laschen angeformt sind.

Bekannt sind medizinische Verpackungen, bei denen ein flach auf der Packung liegender Deckel zwei seitlich einander gegenüberliegende flache Ränder der Packung umfaßt, so daß Deckel und Unterteil gegeneinander verschoben werden können.

Diese Verpackungen haben u. a. den wesentlichen Nachteil, daß sie nicht kindersicher sind. Um sie zu öffnen und so an das Medikament zu gelangen, benötigt ein Kind die Fähigkeit, einen Bewegungsablauf, nämlich das Verschieben von Deckel und Unterteil zueinander, nachzuvollziehen.

Definitionsgemäß gilt eine Verpackung aber erst dann als kindersicher, wenn mindestens zwei unterschiedliche Bewegungsabläufe zu ihrer Öffnung notwendig sind.

Die bekannten Verpackungen weisen dieses Merkmal nicht auf oder sind so kompliziert und teuer herzustellen und zu gebrauchen, daß ihr Verkauf bzw. ihre Verwendung Schwierigkeiten bereitet.

Den Nachteil der geringen Kindersicherheit weist auch die DE-B-20 13 868 auf, welche zwar in ihrem verkaufsfertigen Zustand Laschen vorsieht, die jedoch vor dem ersten Gebrauch der Packung entlang einer perforierten Linie abgerissen werden. Dadurch kann die Verpackung nicht mehr kindersicher wiederverschlossen werden.

Aus der US-A-29 85 296 ist ein Behälter zur Aufnahme von Tintenpatronen bekannt, welcher einen Deckel aufzeigt, der einen mundförmigen Schlitz trägt. Dieser Schlitz übergreift mit einer Oberlippe eine Stirnkante des Behälters. Der Inhalt des Behälters wird jedoch durch eine Öffnung im Deckel entnommen.

Eine ähnliche Ausgestaltung zeigt die US-A-31 57 280. Hier sind in einem Behälterdeckel jeweils zwei spiegelbildlich angeordnete, mundförmige Schlitze vorgesehen, welche mit ihren Oberlippen sich gegenüberliegende Stirnkanten eines Aufnahmeteils umfassen. Diese Anordnung hat u. a. den Nachteil, daß beim Wiederverschließen Teile des Deckels abgebogen und zumindest eine Oberlippe über eine Stirnkante gelegt werden muß. Da dieser zusätzliche Handgriff meist unterlassen wird, kann die Verpackung nicht als kindersicher bezeichnet werden.

Der Erfinder hat sich zum Ziel gesetzt, eine Verpackung der obengenannten Art zu entwikkeln, welche absolut kindersicher und leicht und billig herzustellen ist.

Die Aufgabe wird dadurch gelöst, daß zwischen Lasche einerseits und Aufnahmeteil bzw. Deckel anderseits wenigstens eine Schlitzung angeordnet ist, deren Tiefe zumindest der Tiefe der Nut entspricht.

Diese so gestaltete Verpackung hat den Vorteil, daß zu ihrem Öffnen zwei unterschiedliche Bewegungsabläufe der menschlichen Hände notwendig sind, die gleichzeitig ausgeführt werden müssen. Eine Hand beseitigt die mittels der Laschen erzeugte Begrenzung der relativen Bewegung zwischen Deckel und Aufnahmeteil, die andere führt diese relative Bewegung, welche in einer Verschiebung des Deckels am Aufnahmeteil mittels in seitlichen Nuten eingreifende Führungsstreifen besteht, aus.

Diese Bewegungsabläufe kann ein Kind nicht gleichzeitig vollziehen, weshalb der Verpackungsbehälter kindersicher ist und besonders zur Aufnahme von Medikamenten oder Medikamentenbehältnissen dient.

Durch die Herstellung der Lasche aus elastischen bzw. reversiblem Werkstoff wird vor allem die Wiederbenutzung des Behälters erleichtert. Außerdem braucht der Benutzer keine zusätzlichen Handgriffe zum Verschließen der Packung vorzunehmen; diese schließt sich selbständig nach dem Verbringen des Deckels in Schließlage.

Durch die Schlitzung zwischen der Lasche einerseits sowie dem Aufnahmeteil bzw. dem Deckel anderseits — bevorzugt als Rand- oder Sackschlitz ausgebildet — wird es möglich, die Lasche so weit abzubiegen, daß der Deckel ohne Schwierigkeiten über die Lasche weggeschoben werden kann, dies selbst dann, wenn er eigene Randnuten aufweist.

Erfindungsgemäß weist zudem der Sack- oder Randschlitz einen Fußpunkt auf, von dem eine die Lasche querende Soll-Bruchlinie oder Knicklinie ausgeht, welche von der Lasche Endstücke abteilt sowie gegebenenfalls von einer Verstärkungsrippe überlagert ist. Die Verstärkungsrippe dient u. a. der besseren Reversibilität der Endstücke bzw. zur Unterstützung von deren Rückstellkraft.

Einer weiteren Verbesserung der Sicherung des Verpackungsbehälters dienen an den Endstücken angeformte Vorsprünge oder quer zur Schubrichtung des Deckels verlaufende Querstege; sie verhindern ein Übergleiten des Deckels über die Lasche. Auch dieses Hindernis wird durch Herabbiegen der Laschenendstücke beseitigt.

Bei einem Verpackungsbehälter, welcher sowohl am Deckel als auch am Aufnahmeteil — miteinander korrespondierende — Laschen aufweist, hat es sich als günstig erwiesen, an der einen Lasche Vorsprünge anzuformen, welche in Durchbrüche in der benachbarten Lasche eingreifen. Dabei genügt es, wenn zum Öffnen eine Lasche gebogen wird.

Vorzugsweise wird beim Herstellen des Verpackungsbehälters mit Inhalt bzw. nach dem Füllen des Verpackungsbehälters dieser mit einem Siegel an oder in den Schlitzen versehen. Ein ungebrochenes Siegel ist als Zeichen für eine ungebrauchte Packung zu werten.

Dieser so gestaltete Verpackungsbehälter ist denkbar einfach, aber wirksam, und sehr kostengünstig herzustellen.

Weitere Vorteile, Merkmale und Einzelheiten der Erfindung ergeben sich aus der nachfolgenden Beschreibung bevorzugter Ausführungsbeispiele sowie an Hand der Zeichnung; diese zeigt in:

Fig. 1 einen geschlossenen Verpackungsbehälter in Schrägsicht mit einer Aufnahmemulde,

Fig. 2 einen etwa zur Hälfte geöffneten Verpackungsbehälter in Schrägsicht,

Fig. 3 eine Ausführungsform eines Verpackungsbehälters entsprechend Fig. 1 mit zwei Aufnahmemulden,

Fig. 4 eine weitere Ausführungsform eines Verpackungsbehälters nach Fig. 1,

Fig. 5 eine weitere Ausführungsform eines Verpackungsbehälters nach Fig. 1,

Fig. 6 eine weitere Ausführungsform eines Verpackungsbehälters.

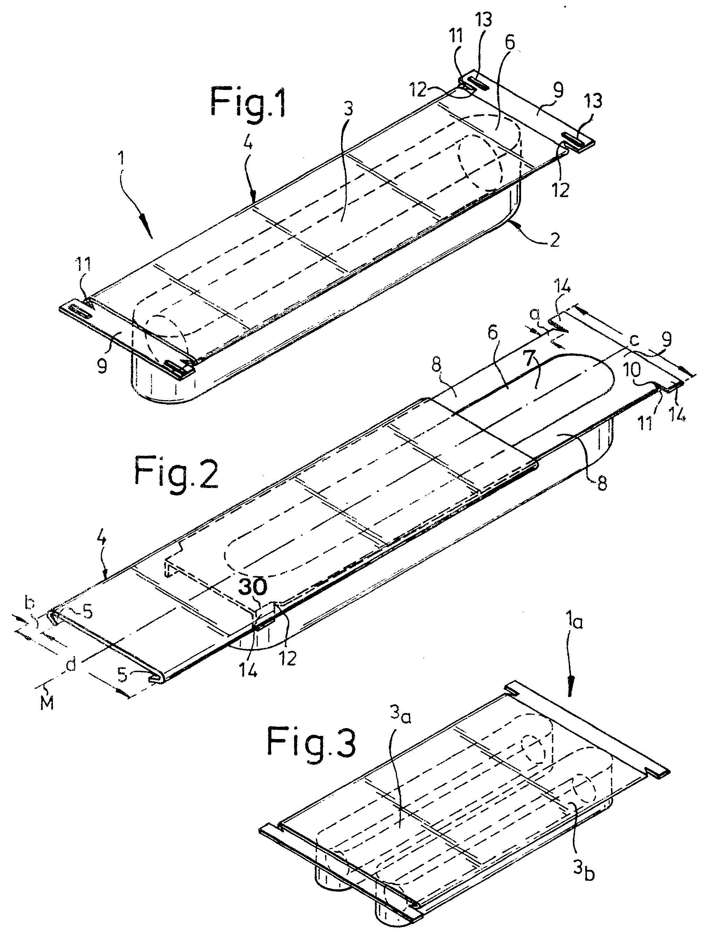

Ein Verpackungsbehälter 1 weist gemäß Fig. 1 einen Aufnahmeteil 2 für Medikamente, Medikamentenröhrchen 3 oder dergleichen und einen rechteckigen Deckel 4 auf.

Die beiden Längsseiten des Deckels 4 sind in ihren Randbereichen so zur Mittelachse M des Deckels 4 umgebogen, daß zwei — deutlich in Fig. 2 gezeigte — Nuten 5 entstehen.

Der Aufnahmeteil 2 besteht aus einer rechteckigen Platte 8, deren Umriß etwa dem des Deckels 4 entspricht. In diese Platte 8 ist eine Öffnung 6 eingeformt, an die eine Aufnahmemulde oder ein Behälter 7 zur Aufnahme eines Medikaments oder Medikamentenröhrchens 3 anschließt.

An die beiden Stirnseiten der Platte 8 sind Laschen 9 angeformt.

Am Übergang von der Lasche 9 zur Platte 8 ist im Randbereich ein Sack- oder Randschlitz 10 mit einer Länge a ausgebildet, welche zumindest der Tiefe b der Nut 5 entspricht. Dadurch entstehen an der Lasche 9 Endstücke 14. Der Sackschlitz 10 ist gegebenenfalls mit einem in Fig. 2 angedeuteten Siegel 11 versehen, das beim erstmaligen Gebrauch des Verpackungsbehälters 1 gebrochen werden muß.

Die Laschen 9, insbesondere aber die Endstücke 14, bestehen aus flexiblem bzw. elastischem Material.

In Schließlage überdeckt der Deckel 4 die Öffnung 6, wobei die Nuten 5 die beiden Längsseiten der Platte 8 umgreifen und die Lage des Deckels 4 von den Laschen 9 der Platte 8 bestimmt ist, deren Länge c größer ist als die Breite d des Deckels 4; durch diese Ausbildung wird der Deckel 4 in Schließlage festgehalten (Fig. 1).

Zum Öffnen des Behälters 7 müssen die Endstücke 14 der Lasche 9 beidseitig nach unten gebogen werden (siehe gestrichelte Linie in Fig. 2), wobei der Fußpunkt 12 des Sackschlitzes 10 eine Knicklinie 30 bestimmt. Damit wird der Deckel 4 freigegeben und kann mit der anderen Hand über die Lasche 9 hinweggeschoben werden.

Bei einem nicht verdeutlichten Ausführungsbeispiel des erfindungsgemäßen Verpackungsbehälters 1 kann die Lasche 9 aber auch so ausgebildet sein, daß sie zur Freigabe des Deckels 4 insgesamt nach unten gebogen werden muß.

Zur Verbesserung der Reversibilität der Lasche 9 bzw. der Rückstellfähigkeit ihrer Endstücke 14 können an diese über den Knicklinien 30 Rippen 13 angeformt sein.

Fig. 3 zeigt einen Verpackungsbehälter $1_a$, welcher dem in Fig. 1 wiedergegebenen entspricht, der jedoch der Aufnahme von zwei Medikamentenröhrchen $3_a$ und $3_b$ oder dergleichen in zwei Behältern 7 dient.

Vorgesehen ist auch, die Öffnung/en 6 mit einer — nicht dargestellten — Folie luftdicht abzuschließen, welche dann vor dem erstmaligen Gebrauch des Verpackungsbehälters 1 entfernt werden muß.

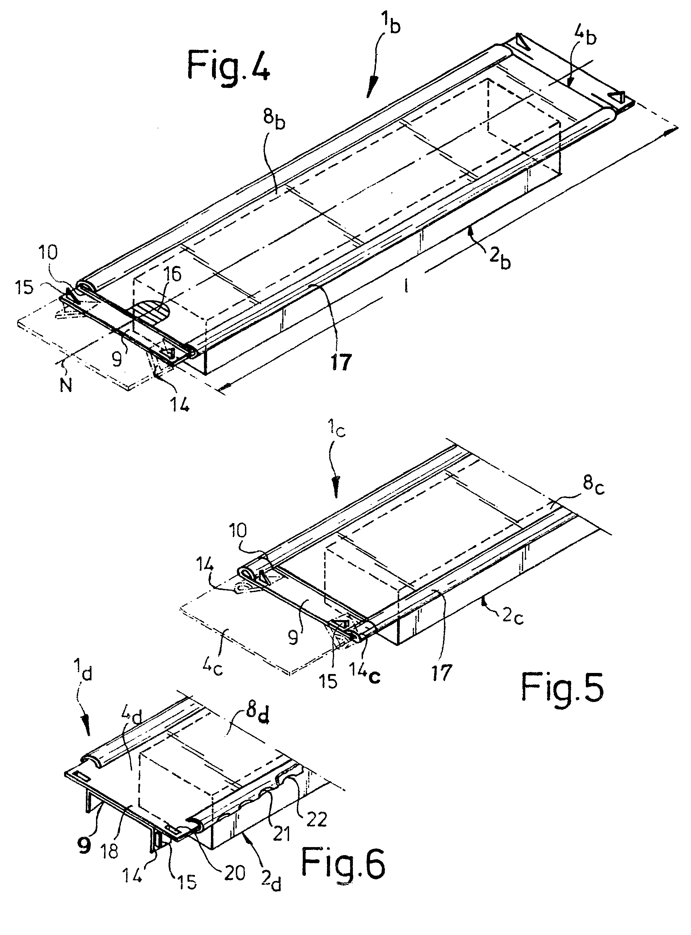

Die Fig. 4 bis 6 zeigen jeweils einen Verpackungsbehälter $1_b$, $1_c$ bzw. $1_d$ mit unterschiedlicher Ausführung der Verschlußorgane.

Während in Fig. 1 der Deckel 4 die Nuten 5 aufweist, welche die Platte 8 des Aufnahmeteils 2 umgreifen, ist bei den Ausführungsbeispielen des Verpackungsbehälters $1_b$ bzw. $1_c$ in Fig. 4 bzw. 5 der Deckel $4_b$ bzw. $4_c$ jeweils als eine einfache rechteckige Platte ausgebildet, welche in Nuten 17 der Platte $8_b$ bzw. $8_c$ des Aufnahmeteils $2_b$ bzw. $2_c$ eingeschoben wird. Die Nuten 17 sind an der Platte $8_b$ bzw. $8_c$ durch Umbiegung der Randbereiche zur Mittelachse N hin hergestellt worden.

An die beiden kurzen Stirnseiten der Platte $8_b$ bzw. $8_c$ sind jeweils die Laschen 9 aus flexiblem bzw. elastischem Material angeformt. Auch bei diesen Ausführungsformen erzeugen die Sackschlitze 10 zwischen den Laschen 9 und den Platten $8_b$ bzw. $8_c$ jene Endstücke 14, welche hier allerdings jeweils mit einem Quersteg oder Höcker 15 versehen sind.

Durch Herabbiegen beider Endstücke 14 einer der Laschen 9 werden jene Höcker 15 aus der Schubbahn des Deckels $4_b$ bzw. $4_c$ genommen, so daß letzterer verschoben werden kann.

Die Ausführungsbeispiele nach Fig. 4 und 5 unterscheiden sich vor allem dadurch, daß bei Fig. 5 die Endstücke 14 der Lasche 9 eine — entsprechend dem benachbarten Nutenbereich — zur Mittellachse N hin gekrümmte Randzunge $14_c$ aufweisen.

Zur besseren Handhabung des Deckels $4_b$ in Fig. 4 ist an diesem eine Griffmulde 16 oder dergleichen vorgesehen.

Beim Ausführungsbeispiel der Fig. 6 sind bei einem Verpackungsbehälter $1_d$ nicht nur an die Stirnseite des Aufnahmeteils $2_d$ Laschen 9 angeformt, sondern auch der Deckel $4_d$ weist an beiden Enden Laschenstreifen 18 auf. In jedem Laschenstreifen 18 sind Durchbrüche 20 vorgesehen, in welche in Schließlage des Verpackungsbehälters $1_d$ Querstege oder Höcker 15 der anliegenden Lasche 9 eingreifen.

Auch bei diesem Ausführungsbeispiel müssen zum Öffnen des Verpackungsbehälters $1_d$ durch die Sackschlitze 10 an der Lasche 9 gebildete Endstücke 14 abgebogen werden, so daß die Querstege oder Höcker 15 aus den Durchbrüchen 20 entfernt sind.

In Fig. 6 ist auch gezeigt, daß — vorzugsweise bei Tablettenpackungen — der Aufnahmeteil $2_d$ bzw. dessen Platte $8_d$ zum Deckel $4_d$ gerichtete Ausformungen 21 aufweist, welche mit Einformungen 22 im Deckel $4_d$ etwa übereinstimmen. Beim Verschieben des Deckels $4_d$ rasten in dessen Einformungen 22 — bevorzugt nach jeder Tablette oder dergleichen — die Ausformungen 21 des Aufnahmeteils $2_d$ ein, so daß die nachfolgende Tablette oder dergleichen geschützt bleibt.

**Patentansprüche**

1. Verpackungsbehälter mit einem Aufnahmeteil (2) für Füllgüter, insbesondere für Medikamente oder dergleichen, welche gegebenenfalls in einem Medikamentengefäß untergebracht sind, und einem Deckel (4), der mittels in seitliche Nuten (5, 17) eingreifender Führungsstreifen am Aufnahmeteil verschiebbar ist, wobei an einander gegenüberliegenden Enden des Aufnahmeteils (2) und/oder des Deckels (4) die Verschiebbarkeit des Deckels (4) verhindernde, seitlich überstehende und abbiegbare Laschen (9, 18) angeformt sind, dadurch gekennzeichnet, daß zwischen Lasche (9 bzw. 18) einerseits und Aufnahmeteil (2) bzw. Deckel (4) anderseits wenigstens eine Schlitzung (10) angeordnet ist, deren Tiefe (a) zumindest der Tiefe (b) der Nut (5, 17) entspricht.

2. Verpackungsbehälter nach Anspruch 1, dadurch gekennzeichnet, daß die als Sack- oder Randschlitz (10) ausgebildete Schlitzung einen Fußpunkt (12) aufweist, von dem eine die Lasche (9, 18) querende Soll-Bruchlinie oder Knicklinie ausgeht, welche von der Lasche Endstücke (14) abteilt und gegebenenfalls von einer Verstärkungsrippe (13) überlagert ist.

3. Verpackungsbehälter nach Anspruch 1 oder 2, dadurch gekennzeichnet, daß die Schlitzung (10) durch ein Siegel (11) verschlossen ist.

4. Verpackungsbehälter nach wenigstens einem der Ansprüche 1 bis 3, dadurch gekennzeichnet, daß auf den Endstücken (14) Vorsprünge oder quer zur Schubrichtung des Deckels (4) bzw. Aufnahmeteils (2) verlaufende Querstege (15) angeordnet sind.

5. Verpackungsbehälter nach wenigstens einem der Ansprüche 1 bis 4, dadurch gekennzeichnet, daß die auf der Lasche (9 oder 18) des Aufnahmeteils (2) oder des Deckels (4), bevorzugt auf den Endstücken (14) der Lasche/n, angebrachten Vorsprünge oder Querstege (15) in Schließlage des Verpackungsbehälters (1) in Durchbrüche (20) oder dergleichen Organe der Lasche (18 oder 9) des jeweils anderen Behälterteils (4 oder 2) eingreifen.

**Claims**

1. Packaging container with recepticle part (2) for contents, in particular for medicaments or the like which if desired or necessary are contained in a medicament vessel, and a lid (4) which can be moved by sliding in grooves (5, 17) at the sides of the recepticle part, at the opposite ends of said recepticle part (2) and or the lid (4) there being tongues (19, 18) which project out at the sides, can be bent over, and which hinder the sliding of the lid (4), in which between said tongue (9 or 18) on the one hand and the recepticle (2) or lid (4) on the other a slit (10) is provided, the depth (a) of which slit being at least as great as the depth (b) of the groove (5, 17).

2. Packaging container according to claim 1, in which the slit which is in the form of a notch or edge slit (10) features an end point (12) from which an intented fracture or bending line runs across the tongue (9, 18) to divide off end pieces (14) from the rest of the tongue, if necessary with superimposing strengthening ribs (13).

3. Packaging container according to claim 1 or 2, in which the slit (10) is closed off by a seal (11).

4. Packaging container according to at least one of the claims 1 to 3 in which projections or struts (15) running transverse to the direction of sliding of the lid (4) or recepticle part (2) are proveded on the end pieces (14).

5. Packaging container according to at least one of the claims 1 to 4, when the said container is in the closed position, the projections or transverse struts (15) on the tongue (9 or 18) of the recepticle (2) or lid (4) — preferably on the end pieces (14) of the tongue/tongues — engage in openings (2a) or the like organs in the tongue (18 or 9) of the other part (4 or 2) of the container.

**Renvendications**

1. Boite d'emballage avec compartiment pour produits (2), en particulier pour médicaments ou produits semblables lesquels dans ce cas sont placés dans un recipient à médicaments, et un couvercle (4) qui au moyen de canelures de guidage coulisse dans des rainures latérales (5, 17), et qui présente aux extrémités opposées du compartiment (2) et/ou du couvercle (4) des pattes rabattables (9, 18) en saillie qui permettent d'éviter le déplacement du couvercle, caractérisé par le fait, qu'entre les pattes (9, 18) d'une part et le compartiment (2), respectivement le couvercle (4) d'autre part, se trouve une entaille (10) d'une profondeur (a) au moins égale à la profondeur (b) de la rainure (5, 17).

2. Boite d'emballage d'après la revendication 1, caractérisée par le fait, que l'entaille (10) ainsi formée présente une base (12) d'où une ligne de pliage ou de cassure traversant la patte se propage, séparant l'extrémité de la patte (14) renforcée dans ce cas par une nervure (13).

3. Boite d'emballage d'après les revendications 1 ou 2, caractérisée par le fait, que l'entaille (10) est scellée à l'aide d'un cachet (11).

4. Boite d'emballage d'après au moins une des revendications 1 à 3, caractérisée par le fait que sur l'extrémité de la patte (14) perpendiculaire à la direction de glissement du couvercle (4) respectivement du compartiment (2) se trouve un téton ou un ergot (15).

5. Boite d'emballage d'après au moins une des revendications 1 à 4, caractérisée par le fait, que sur la patte (9 ou 18) du compartiment (2) ou du couvercle (4), les tétons ou ergots (15) se trouvant de préférence sur l'extrémité de cette ou de ces pattes (14) se clippent, lorsque l'emballage est en position fermée, dans la fenêtre (20) avec chaque fois la partie correspondante de la boite (4 ou 2).

Fig.1

Fig.2

Fig.3

Fig.4

Fig.5

Fig.6